# EUROPEAN PATENT SPECIFICATION

(11) **EP 0 945 727 B1**
(45) Date of publication and mention of the grant of the patent: **11.06.2003**
(21) Application number: 99302242.5
(22) Date of filing: 23.03.1999
(51) Int. Cl.: G01N 33/50, G01N 33/569

(54) **Method for diagnosing HIV infection**
Verfahren zur Diagnose von HIV-Infektionen
Méthode de diagnostic pour détecter l'infection avec VIH

(30) Priority: 24.03.1998 JP 9687098
(43) Date of publication of application: 29.09.1999
(73) Proprietor: JCR PHARMACEUTICALS CO., LTD., Ashiya, Hyogo 659-0021 (JP)
(72) Inventor: Nishimoto, Akihiro, Tarumi-ku, Kobe, Hyogo (JP); Koga, Jun-ichi, Nishi-ku, Kobe, Hyogo (JP); Shirono, Hiroyuki, Tarumi-ku, Kobe, Hyogo (JP); Kurimura, Takashi, Amagasaki, Hyogo (JP)
(74) Representative: Smart, Peter John

(56) References cited:
- WO-A-90/04180
- HOSSAIN, M. M. ET AL: "Interleukin-9 receptor.alpha. chain mRNA formation in CD8+ T cells producing anti-human immunodeficiency virus type 1 substance(s)" ACTA VIROLOGICA, vol. 42, no. 1, February 1998 (1998-02), pages 47-53, XP002108695
- RENAULD J -C ET AL: "INTERLEUKIN-9" INTERNATIONAL REVIEW OF EXPERIMENTAL PATHOLOGY, vol. 34A, 1 January 1993 (1993-01-01), pages 99-109, XP000616468

## Description

The present invention relates to a novel diagnostic method for infection with the human immunodeficiency virus (HIV).

HIV belongs to the family of retroviruses and possesses the basic structure composed of an external envelope and an internal capsid which contains RNA and reverse transcriptase. HIV has a gene structure which consists of the gag gene, or a structural gene, the pol gene, or a gene encoding the reverse transcriptase, and the env gene, or a gene encoding the envelope glycoprotein, with the LTR (long terminal repeat) nucleotide sequences being contained at the ends thereof to regulate the gene expression.

Referring to the mechanisms of HIV infection and proliferation, HIV infection proceeds firstly through integration of HIV into the cell surface by way of the binding of the envelope glycoprotein to the target cell receptor and is then established by reverse transcription of its RNA into DNA of the target cell with its own reverse transcriptase, while HIV proliferates by transcription to the target cell RNA of HIV's DNA as incorporated into the target chromosome, followed by translation to the viral protein to thereby generate mature HIVs.

The host-specificity in viral infection is determined by membrane-receptor specificity in target cell to the envelope glycoprotein. In particular, HIV infection requires the extracellular domain of lymphocyte CD4 as a glycoprotein being connected with the viral infection specificity, wherein CC-chemokyne receptor, fusin, etc. act as its receptor.

In addition, HIV possesses several types of regulatory genes for its own expression and proliferation, and among such regulatory genes, the tat and rev genes encode individually the Tat and Rev proteins being essential for viral proliferation.

LTR, or the promoter region for HIV, is constructed from the U3, R and U5 regions; the U3 region contains the TATA and CC boxes as well as the NFκB sequence required for proliferation, while the R region accomodates the TAR sequence which, after being transcribed to RNA, assumes the hair-pin structure to bind to the Tat molecule, whereby the binding between the Tat molecule and TAR sequence is considered essential for initiation and continuation of the transcription. It is further added that the Rev protein binds to the RRE sequence of mRNA to thereby function during mRNA splicing.

What is noted in the above has been identified as the fundamental characteristic properties of HIV, which belongs to the family of retroviruses. However, many of the diseases associated with retroviruses are intractable diseases difficult to be treated, and are typically exemplified by the highly lethal, acquired immune deficiency syndrome (AIDS) caused by HIV.

The acquired immune deficiency syndrome (AIDS) caused through HIV infection is characterized by slow depletion or destruction of the immune system which proceeds over a lengthened period of time as long as several to ten several years after infection. Upon onset of primary immune response, the viral infection symptoms disappear gradually, resulting in decreased viral replication; the virus and its replication are hardly detected clinically in peripheral blood monocytes (PBMCs) over the several-year period until the later phase of HIV infection appears.

Among individuals infected with HIV I, the long-term asymptomatic patients, though found in small number, have recently been noted to exist, wherein their lymphocytes are demonstrated to retain CD-positive T cells capable of suppressing in vitro HIV replication.

As is the case with many viral diseases, CD8-positive T lymphocytes are assumed to play a vital role in the defense of the living body against HIV infection, and the significance of CD8-positive T cells has been recognized in the clinical latency of infectious symptoms and long-term asymptomatic patients [J. L. Whitton et al., Reven Press New York, pp. 369-381 (1990), W. E. Paul, Cell, 82; 177 (1995)]. In particular, it is discovered that the activated CD8-positive T cells originated from the peripheral blood of HIV-infected patients secrete several soluble HIV-suppressory factors, thus contributing to prevention of in vitro infection [J. E. Brinchman et al. J. Immunol., 144, 2961 (1990), C.E. Heckewicz et al., AIDS Res. Hu. Retroviruses, 8: 1039, (1992)].

The supernatant of culture broth of CD8-positive T cells originated from HIV-infected patients shows increased levels of RANTES (6 to 95 ng/ml), MIP-la (20 to 255 ng/ml) and MIP-1 b (37 to 191 ng/ml), and such HIV-suppressory factors are found to inhibit cellular infection with HIV [Fiorenza Cocchi et al., Science, 270: 1811 (1995)].

As is explained in the above, the CD8-positive T lymphocytes from the HIV-infected individuals secrete diverse cytokines, suggesting that T lymphocytes are kept in the activated state.

CD8 antigen of cytolytic T lymphocytes, which is a glycoprotein with a molecular weight of 32 kDa belonging to the immunoglobulin superfamily, is confirmed to be involved in the recognition of major histocompatibility (MHC-1) antigens. On the other hand, helper T lymphocytes (Th) possess CD4 antigen with a molecular weight of 55 kDa. These subset antigens of CD8 and CD4 lymphocytes are assumed to be deeply involved in the regulation of cellular activation.

It has been demonstrated that human CD4-positive lymphocytes, when stimulated through activation by anti-CD3 antigen, express mRNA for interleukin 9 (IL-9), resulting in secretion of IL-9 [Renauld, J.C. et al., J. Immunol. 144:4235 (1990)]. IL-9 acts as a growth factor for T helper lymphocytes and consequently participates in the activation of CD4-positive T cells (Proc. Acad. Sci. USA, 85:8934 (1989)]. In the case of human lymphocytes, autocrine growth and activation have been suggested for the ligands for cytokines inclusive of IL-9 and the ligand receptors. IL-9 is generated in vitro by CD-positive T cells of activated type II T-lymphocytes (Th2) as well as naïve CD4-positive cells [J. Immunol. 147:3848 (1991)].

IL-9, whose gene is composed of five exons and four introns, has been identified as being capable to support the growth of helper T cells, and, because of the diversity of its target cells, constitutes a multifunctional cytokine concerned in mast cells, megakaryoblastic leukaemia, fetal thymocytes, murine erythroid progenitors and human erythroid progenitors.

IL-9 Receptor (IL-9R), whose gene is composed of nine exons and eight introns [Blood, 83:3199 (1944)], belongs to the superfamily of hemopoietin receptors, and exerts interaction with the γ-chain of interleukin 2 receptor (IL-2R) for the signal transduction [Renauld J-C, et al., J. Leuko. Biol., 57: 353 (1995)].

IL-9 response to T lymphocytes is known to change markedly according to the type and timing of antigen stimuli. Fetal thymocytes exhibit growth under specific combinations of IL-2 with IL-9. Expression of IL-9 is restricted to activated T-cells, and dynamic analysis of its induction reveals that expression of IL-9 mRNA appears in the later phase (its peak reached at 28 hours) of T-cell activation and requires mediation by IL-2. As is shown by Western blotting analysis, stimulation of polynucleated leukocytes with IL-9 yields four unfixed tyrosine phosphorylation bands but does not promote phosphorylation of serine-threonine kinase (Raf-1, MAP).

Interleukin-9 receptor (IL-9R) is present on the cytoplasmic membranes of various cells inclusive of T-cells, while demonstrating its specifically high affinity for IL-9 (Kd=∼100 pM) [Idzerda, R.J. et al., J. Exp. Med., 171:961 (1990)]. In humans, IL-9R messenger RNA shows heterogeneity owing to its alternative splicing mechanism. In the human genome, there exist at least four false genes for IL-9R, which genes each exhibit about 90% homology with IL-9R. Generally, the cytokine receptors consists of two chains, out of which the first chain is specific to the ligand type, with the additional chain being common to several types of cytokines; the γ-chain of IL-2R is connected with IL-9R, and the antibody against the γ-chain inhibits entirely the activity of IL-9R.

Consequently, it has been found that IL-2 and IL-9 elicit intensified synergetic effect against the target cell, and furthermore, IL-9 in some cell lines can act to protect cells from apoptosis rather than to proliferate cells, whereas IL-2 is effective for cellular proliferation. In view of the facts that apoptosis is a phenomenon uniquely characteristic to the activated cells and that IL-9 in the form of activated lymphocyte exhibits anti-apoptotic property, IL-9 is assumed to play a vital role in apoptosis.

Human T lymphocyte cell lines and their clones, whether they are of CD4-positive or CD8-positive type, express enhanced IL-9R messenger RNA in response to IL-9 to show cellular proliferation. For example, tumour-specific, CD8-positive T-lymphocytes express the messenger RNA of IL-9R, whereby IL-9 promotes cellular proliferation of survival prolongation. In order to elicit the optimal effect of IL-9, the cell pre-activation is required [Herederic A.H. et al., J. Immunol., 150:2634 (1993)]. M.M. Hossain et al., Acta virologica, 42:47-52, 1998 discloses a search for genes associated with anti-HIV-1 activity of CD8+ T cells using molecular cloning. The relation between the anti-HIV activity of CD8+ T cells and the IL-9R-α mRNA expression from the cDNA clones obtained was examined. IL-9R-α mRNA was assayed by RT-PCT. Of five cases showing high level of anti-HIV-1 activity, the mRNA was detected in four cases. Of ten cases showing low level of anti-HIV-1 activity, the mRNA was detected in one case.

The present invention aims at providing a diagnostic method for detecting HIV infections.

The present inventors, taking notice of the fact that the lymphocytes from HIV-infected, long-term asymptomatic patients retain CD8-positive lymphocytes exhibiting anti-HIV activity, conducted genome analysis of such lymphocytes.

From CD8-positive T lymphocytes from each of the individuals infected and not infected with HIV, cDNA libraries were constructed and screened into subsets with mRNA of CD8-positive T cells of the non-infected person, and utilising a probe specific to the remaining CD8-positive T cells of the HIV-infected person, there was obtained a colony having cDNA of CD8-positive T cells characteristic of the HIV-infected person (Table 1 and 2). Hybridisation was repeated twice, and the resulting positive colony was determined for nucleotide sequence, followed by screening for homology, leading to the discovery that IL-9R was markedly expressed.

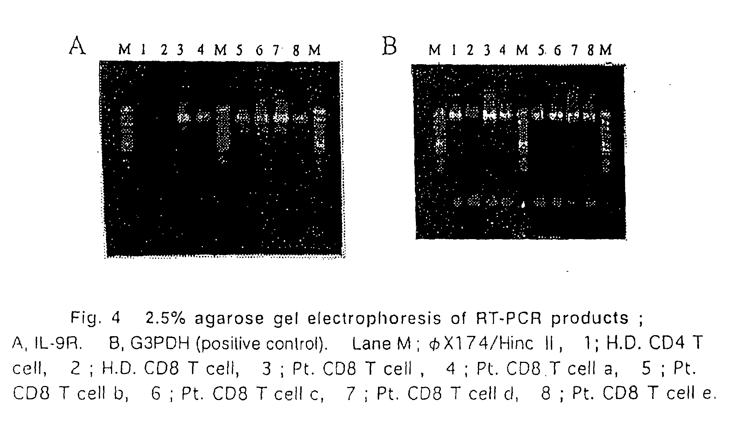

Though the level of expression showed individual variations among patients, it was found that the more potent the anti-HIV activity exhibited by the patient-derived CD8-positive T lymphocytes, the higher the relative degree of expression of IL-9R (Fig. 4 and Table 5). Expression of IL-9R in CD8-positive T lymphocytes is characteristic to the HIV-infected, long-term asymptomatic patients, whereas expression of IL-9R in non-infected individuals cannot be detected or is detectable only at a negligible level (Fig. 4 and Table 5).

Consequently, the present inventors conducted genomic analyses to characterise anti-HIV activity of the long-term asymptomatic HIV patients in terms of expression of IL-9R, thus permitting the patients infected with viruses inclusive of HIV to be diagnosed.

The present invention has been completely based on such findings and relates to a diagnostic method for detecting HIV infection, characterized in that said method comprises utilizing as a specific index of infection a T-cell growth factor receptor (IL-9R) which expresses specifically in T-cell lymphocytes.

The T-cell lymphocytes are preferably either CD4-positive or CD8-positive lymphocytes, and supernatants of their cultures can be used as a sample in the diagnosis.

The present inventors now have clarified that the T lymphocytes from the peripheral blood of HIV-infected, long-term asymptomatic patients are kept in the activated state at the genetic level. Since IL-9R is expressed as an index of lymphocyte activation, addition of ligands to IL-9R enables the anti-viral activity of lymphocytes to be enhanced or modified. Such ligand proteins and polypeptides include, for example, cytokines and chemokines, wherein IL-2 and IL-9 showing enhanced affinity for IL-9R are assumed to be efficient and are especially considered effective as an AIDS therapeutic agent. This furthermore means that any substances capable of inducing expression of IL-9R would constitute an anti-HIV drug, as well.

Although IL-2 is known to be essential for in vitro cultivation of lymphocytes, it may be possible to activate lymphocytes by simultaneous or individually separate administration of IL-1 and IL-9. Also, non-proteinaceous substances can be used as a ligand for IL-9R to activate lymphocytes. Furthermore, the ligands for IL-9R may be polypeptides containing the amino acid sequence of the binding site for IL-2 and IL-9 receptors.

In addition, the modifying and activating agonists for the activation of lymphocytes include IL-9 homologues showing high affinity for IL-9R, and preparation of such homologues may be easily feasible by known genetic engineering procedures such as replacement, deletion and addition for amino acids.

By way of a means for screening an agonist or antagonist for IL-9R on the cellular membrane of lymphocytes, CD8-positive T lymphocytes were isolated and cultivated from the peripheral blood of the HIV-infected, long-term asymptomatic patients, and as an assay for the agonist or antagonist activity, use may be made of measurement of changes in pH with a cytosensor to determine metabolic activity of cells as well as LTR-CAT and RT analyses in the form of anti-HIV analysis. The terms "agonist" and "antagonist" are understood to comprehend protein/polypeptides and non-proteinaceous substances.

The present invention relates to a diagnostic method for detecting HIV infection, consisting of detecting or measuring expression by CD8⁺ T-cell lymphocytes of the T-cell specific interleukin-9 receptor.

The method may consist of detecting or measuring the production of mRNA coding for IL-9R in a culture of CD8⁺ T-cells.

Alternatively, the method may consist of detecting or measuring the effect of CD8⁺ T-cell expression products on a test system responsive to IL-9R.

Preferably, said test system is the IL-9R mediated suppression of HIV replication in T-cells.

Preferably, said expression products are introduced into said test system as the supernatant from a culture of CD8⁺ T-cells.

Preferably, said method is diagnostic of infection in a long-term asymptomatic HIV infected individual.

Below described are the examples to illustrate the present invention in more detail, while referring to the drawings wherein:

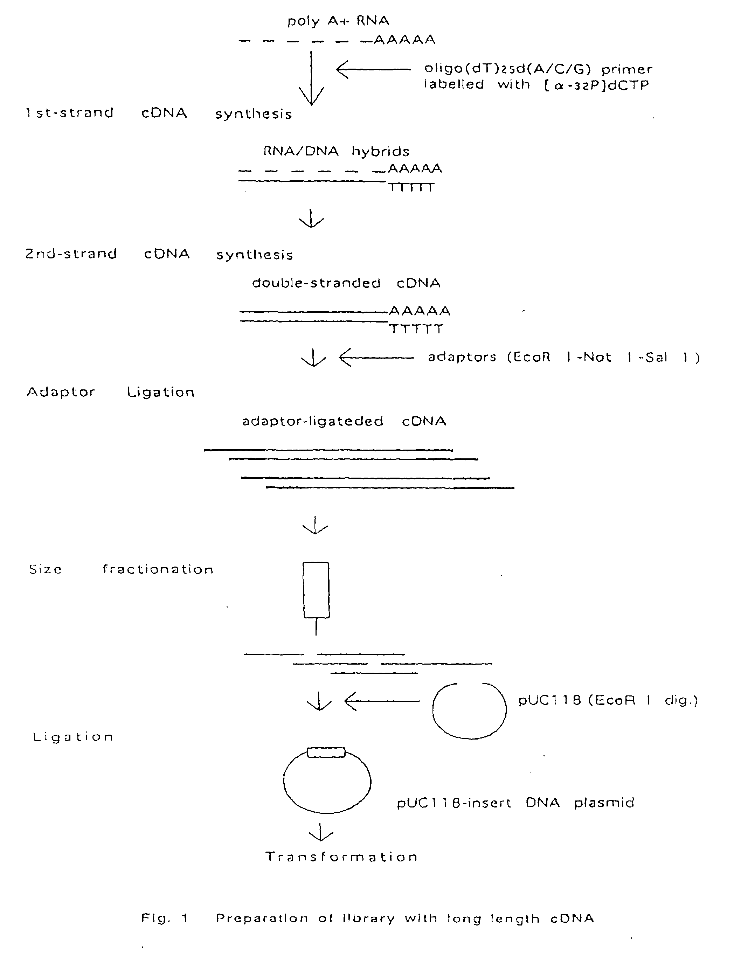

Fig.1 is a schematic illustration of the procedural steps for constructing a cDNA library of CD8-positive T cells from an HIV-infected person, in which, after isolation of mRNA from CD8-positive T cells of an HIV-infected person, reverse transcription was conducted with Oligo (dT) 25d (A/C/G) Primer to give the first-strand DNA; then, the second-strand cDNA was synthesised with RNA depolymerisation enzyme H (supplied by Crontec), DNA polymerase I from E. coli (supplied by Crontec) and DNA ligase from E. coli, (supplied by Crontec). Such ends were bound to an adapter containing the EcoRI, NotI and SaII recognition sites, followed by phosphorylation, and cDNAs with a length of 500 bp or more were recovered by a cDNA fractionation column, then digested with EcoRI and incorporated into pUC 188 at the EcoRI cutting site, followed by transformation of E. coli with the recombinant plasmid by the Hanahan method.

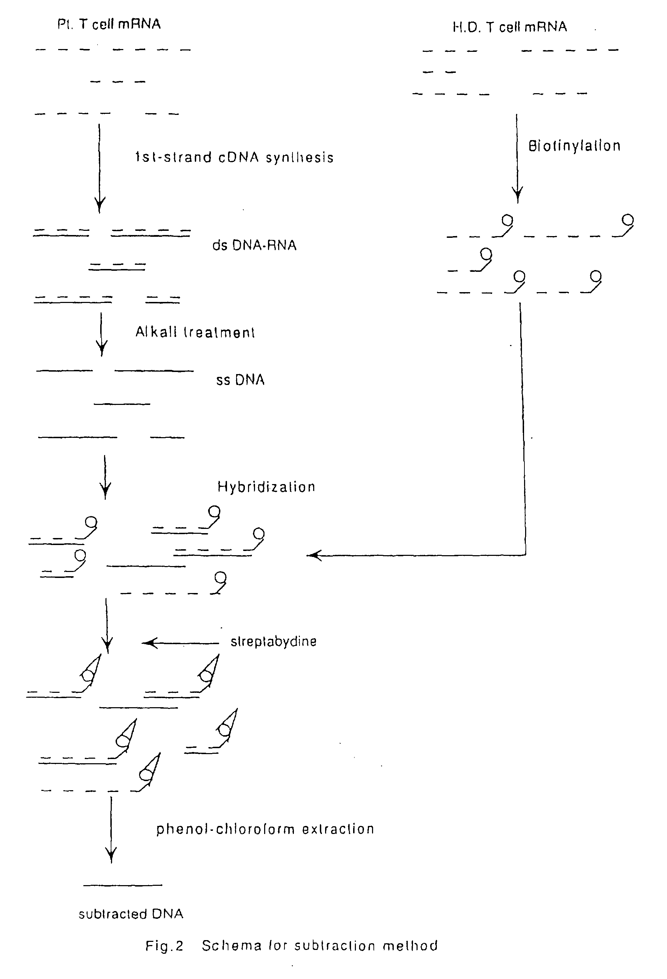

Fig. 2 illustrates schematically the procedural steps for constructing cDNA specific to the HIV-infected, long-term asymptomatic CD8-positive T cells in accordance with the subtraction method, in which mRNA (H.D.T. cell mRNA) from non-infected CD8-positive T cells was isolated and biotinylated, whereas mRNA (Pt.T.cell mRNA) from HIV-infected CD8-positive T cells was isolated, followed by reverse transcription with Oligo(dT)n Primer, and the resultant first-strand DNA (dsDNA-RNA) was subjected to alkali treatment to yield single-strand cDNA (ssDNA); the previously mentioned biotinylated mRNA from healthy CD8-positive T cells and cDNA from HIV-infected CD8-positive T cells were mingled to undergo hybridization, and after addition of streptabydine for binding to biotin, phenol/chloroform solution (1 : 1 mixture) was added to the hybrid, followed by stirring and centrifugation to allow separation into the aqueous and organic layers (phenolchloroform extraction), whereby there was separated out only the aqueous layer containing cDNA (subtracted DNA) specific to the HIV-infected CD8-positive T cells.

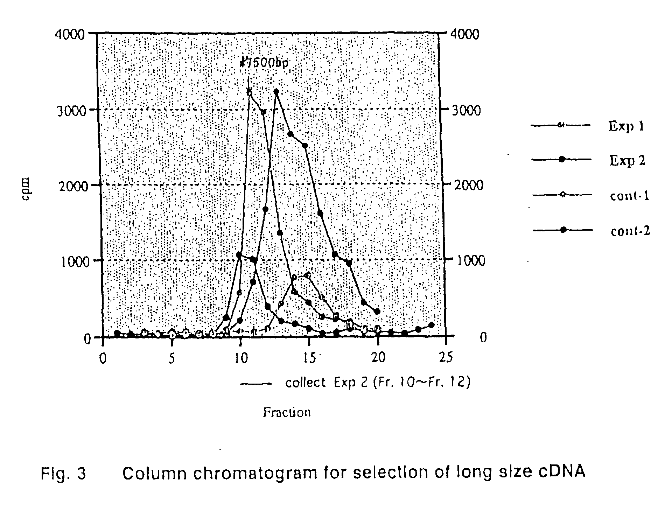

Fig. 3 shows the results of cDNA fractionation column chromatography to select the desired chain length of cDNA in examples, in which chromatographic procedure, after synthesis of the second-strand cDNA from the first-strand cDNA from HIV-infected CD8-positive t cells, cDNA having an adapter bound thereto was poured to a cDNA fractionation column, followed by development with STE buffer (0.5 mM EDTA. 30 mM sodium chloride, 10 mM Tris hydrochloride buffer, pH 7.8); On the basis of the results coupled with those of chromatography for a control, there were recovered fractions containing cDNA with a length of 500 bp or more.
Fig. 4 contains photographs of agarose-gel electrophoretic separations of the RT-PCR products obtained with a primer set for IL-9R in Example 4, in which, using mRNAs from HIV-infected CD8-positive T cells, non-infected CD8-positive T cells and CD4-positive T cells as well as a primer set for IL-9R, the technique of RT-PCR (which involves synthesizing the first-strand cDNA and conducting PCR with the same as a template) was carried out, and the products were subjected to 2.5 % agarose gel electrophoresis (electrophoretic buffer; TAE buffer (40 mM Tris acetate buffer, 1 mM EDTA, pH 8.3)(A); in order to confirm that the RT-PCR technique worked normally, a primer set for G3PDH gene, or a housekeeping gene, was used separately as a positive control (B).
In connection to Fig. 4, the following explanatory notes are given:
Agarose gel, IL-9R; Interleukin-9 receptor.
G3PDH; a housekeeping gene (a gene being constitutively expressed in all the cells)
H.D. CD4 T cell; Healthy or non-infected CD4-positive T cell.
H.D. CD8 T cell; Healthy or non-infected CD8-positive T cell.
Pt. CD8 T cell; HIV-infected CD8-positive T cell.
Lane M; φX174/HincII Maker
Lane 1: H.D. CD4 T cell
Lane 2; H.D. CD4 T cell
Lane 3; Pt. CD8 T cell
Lane 4; Pt. CD8 T cell a
Lane 5; Pt. CD8 T cell b
Lane 6; Pt. CD8 T cell c
Lane 7: Pt. CD8 T cell d
Lane 8; Pt. CD8 T cell e

### Materials used in examples:

Quick prep mRNA Micro purification Kit (supplied by Pharmacia), Great Length cDNA synthesis kit (supplied by CLONTEC), "α-32P" dCTP (3,000 Ci/mmol) (supplied by Amersham), Ligation Kit Ver. 2 (supplied by TaKaRa), pUC118 (supplied by TaKaRa) <EcoRI site digestion, BAP treated>, Max efficiency DH5α competent cell (supplied by GIBNCO), Subtractor Kit (supplied by Invitrogen), Klenow fragment (supplied by TaKaRa), hybond N+Nylon membrane (supplied by Amersham), Whatman filter paper. 3MM (Chr), RPM Kit (Bio 101), Ready.To.Go 1^{st} strand synthesis Kit (supplied by Pharmacia).

### Example 1

### Construction of a cDNA library for CD8-positive T cells of an HIV infected person (Fig. 1, Table 1)

From lymphocytes of an anti-HIV-antibody positive individual, CD8-positive T cells alone were isolated using anti-CD-antibody bound magnetic beads (supplied by Dai-Ichi Kagaku Yakuhin Co.). The CD8-positive T-cells were cultivated over RPMI 1640 culture medium (supplied by Nissui Seiyaku Co.) containing 10% of fetal bovine serum (supplied by Gibco) for several days, and the culture supernatant was added to HIV-infected cells to quantitate the HIV protein p17 in the culture broth with an enzymatic immunological assay kit (supplied by Dynabott). The results showed that a culture broth of CD8-positive T cells from a particularly specified HIV-infected person suppressed markedly production of p17 by HIV infected cells, as compared with the counterparts of CD8-positive T cells originated from other HIV-infected and healthy persons. Then, the CD8-positive T cells which showed anti-HIV activity were cultivated over RPMI culture medium containing 10% of fetal bovine serum for several days, and the cells were recovered to isolate mRNA with use of Quick prep mRNA Micro purification Kit (supplied by Pharmacia), followed by treatment with a reverse transcriptase (supplied by Crontec) utilising Oligo 9dT) 25d (A/C/G) (supplied by Crontec) as a primer to give the first-strand cDNA. By using Oligo (dT) 25d (A/C/G) Primer in place of Oligo (dT)n Primer generally in wide use. annealing was able to be made on the 5' side of the poly A sequence of mRNA to thereby synthesize the protein-encoding first-strand cDNA in the elongated form. After the second-strand cDNA was synthesized with use of RNA depolymerase H (supplied by Crontec) as well as DNA polymerase I (supplied by Crontec) and DNA ligase (supplied by Crontec) both originated from E. coli , their ends were made blunt with DNA polymerase (supplied by Crontec) and then bound to adapters (supplied by Crontec) containing the EcoRI, NotI and SalI recognition sites with use of DNA ligase (supplied by Crontec), followed by phosphorylation with a polynucleotide kinase (supplied by Crontec). During the above-mentioned syntheses of the first-strand and second-strand CDNAS, ³²P-dCTP (supplied by Amersham) was added to the substrates to conduct radiolabeling. In order to select cDNA with about 500 bp or more to meet the requisites as a good-quality cDNA library, the above mentioned cDNAs were applied to a cDNA fractionation column (supplied by Crontec), followed by development with STE buffer (10 mM Tris Hydrochloride buffer of pH 7.8 containing 0.5 mM EDRA and 30 mM sodium chloride). The fractions being equivalent to cDNA with not less than 500 bp were collected and allowed to settle by admixing with twice the volume of ethanol and one-tenth the volume of 3M sodium acetate solution of pH 5.2, followed by recovery by centrifugation. cDNA, as dissolved in 10 µg of TE buffer (10 mM Tris Hydrochloride buffer of pH 8.0 containing 1 mM EDTA), was digested with EcoRI, then isolated by agarose-gel electrophoresis, mingled with dephosphorylated pUC188 vector (supplied by Takara), admixed furthermore with ligase (supplied by Takara) to be incorporated into the EcoRI cutting site of pUC118 (Fig. 3). The resultant plasmid was added to E. coli DH5α competent cells (supplied by Gibco) for incorporation through transformation by the Hanahan procedure. The transformed E. coli was sowed on L-Broth medium containing 1.5 % of agar, and cultivated at 37°C, and the resultant colonies were suspended with L-Broth medium and furthermore diluted with added dimethylsulfoxide to the final concentration of 7 %, followed by storage at -80°C. The library was assumed to show a degree of complexity of 3.6 x 10⁵ cpf (3.9 x 10⁵ cpf/µg).

Table 1 tabulates the recovery results of cDNA from CD8-positive T cell mRNA of an HIV-infected person; 4.0 ng of CD8-positive T cell mRNA of an HIV-infected person was isolated and then subjected to reverse transcription with use of Oligo (dT) 25d (A/C/G) Primer to give 0.4µg of the first-strand cDNA, whereas 5.0 µg of human lymphocyte Burketts' (Daudi) mRNA used as a control generated 2.6 µg of the first-strand cDNA, with the weight ratio of the first-strand cDNA against initial mRNA being shown in % copy in Table 1. Also, the second-strand cDNA was synthesized from the first-strand cDNA, and after being bound with Adapter, cDNA with 500 bp or more was fractionated for recovery by use of a cDNA fractionation column. Such cDNA was used to construct a cDNA library, which was then examined for a specific insert chain length of several ten clones, with the mean values being shown in Table 1, where the terms or wordings have individually the following meanings:
Initial; Amount of mRNA.
Yield ; Amount of the first-strand cDNA
% copy ; Amount of the first strand DNA/the amount of mRNA
Final yield; Amount of cDNA with 500 bp or more fractionated from cDNA fractionation column.
Length (av.): A mean chain length of cDNA in the cDNA library of CD8-positive cells of an HIV-infected person.

**Table 1:**

| Yield of cDNA from mRNA | | |
|---|---|---|
| | Pt.CD8+ T cell | Control |
| Initial | 4.0 µg | 5.0 µg |
| Yield (1st-strand cDNA) | 0.4 µg | 2.6 µg |
| % copy | 11.0 % | 52.5 % |
| Final yield | 0.2 µg | ND |
| Length (av.) | 1,000 bp | < 500 bp |

### Example 2:

### Screening through colony hybridization

### 1) Preparation of a specific probe for CD8-positive T cells of a long-term asymptomatic HIV infected person through the subtraction method.

For the purpose of isolating cDNA which expresses specifically in the CD8-positive T cells showing anti-HIV activity, screening was carried out using as a probe cDNA obtained through the subtraction method (Fig. 2).

From a non-infected person's blood, lymphocytes were isolated by way of centrifugation, and magnetic beads having anti-CD8 antibody bound thereto were allowed to adsorb CD8-positive T cells alone to isolate CD8-positive T cells. Interleukin 2 (IL-2) and phytohemagglutinin (PHA) were added to RPMI 1640 medium containing 10% of bovine fetal serum, followed by cultivation, and the cells were recovered by centrifugation. The CD8-positive T cells originated from a non-infected person were brought into a solution, and mRNA was isolated using Quick prep mRNA Micro-purification Kit (supplied by Pharmacia). Furthermore, photobiotinacetic acid (supplied by Invitrogen) was added, and the mixture was left on standing for 20 min under direct irradiation with a 300W reflector lamp to label mRNA with biotin. Subsequent butanol extraction and ethanol precipitation were conducted to eliminate biotin, and the resultant biotin-free mRNA was labeled with biotin once again; said butanol extraction was repeated until the butanol layer turned transparent, followed by ethanol precipitation to secure complete elimination of biotin.

On the other hand, CD8-positive T cells from an HIV-infected person were isolated and cultivated in a similar manner, and then mRNA was extracted and admixed with oligo dT primer (supplied by Invitrogen) and reverse transcriptase to thereby synthesize the first-strand cDNA. RNA was digested through alkali treatment, and the double-stranded DNA-RNA was converted to single-stranded cDNA. The previously biotin-labeled mRNA from the non-infected CD8-positive T cells was mingled with cDNA from the HIV-infected CD8-positive T cells, followed by heat denaturation to undergo hybridization. After streptavidin (supplied by Invitrogen) was added for bonding to biotin, a phenol/chloroform solution (1:1 mixture) (supplied by Invitrogen) was added, followed by stirring and centrifugation to allow the reaction solution to separate into the aqueous and organic layers, with the aqueous layer alone being separated out. The procedure was repeated several times to remove the cDNA bonded to the biotin-labeled mRNA. In light of the fact that streptavidin turns insoluble through denaturation with phenol/chloroform and stays in the interlayer between the organic and aqueous layers, the cDNA remaining in the aqueous layer was considered to be cDNA having the HIV-infected CD8-positive T cells alone expressed. cDNA specific to an HIV-infected person was admixed with dATP (Supplied by Takara), dGTP (supplied by Takara), dTTP (supplied by Takara), Klenow fragment (supplied by Takara) and 32P-dCTP (supplied by Amersham) to synthesize the second-strand cDNA, which was used as a radiolabelled probe.

### 2) Colony hybridisation

A cDNA library was diluted with L-Broth medium, which was sowed at such a ratio as 500 to 1,000 colonies grow per plate of Broth medium containing 1.5% of agar, followed by cultivation at 37°C. After conclusion of the cultivation, two replica plates were prepared and cultivated at 37°C, then being stored at 4°C. One replica plate was overlaid with a nylon membrane (supplied by Amersham) to transfer the colonies, which was then placed on filter paper (supplied by Wattman) wetted with a denaturation dissolution solution (2 x SSC (0.3M sodium chloride, 0.03M sodium citrate, pH 7.0), 5% SDS) and heated in a microwave oven for about 1 min to expose viral DNA. The membrane was blocked with skimmed milk to undergo hybridisation with a radiolabelled probe. The membrane was washed with each of the washing solutions (Washing solution (1):2xSSC, 1% SDS, pH 7.0; Washing solution (2): 1 x SSC, 1% SDS, pH 7.0; Washing solution (3): 0.1 x SSC, 1% SDS, pH 7.0) and placed on an X-ray film, followed by exposure to light. The colonies hyrbidised with a radiolabelled probe were picked up from another replica plate and sowed in L-Broth medium containing 1.5% of agar, followed by cultivation at 37°C and colony hybridisation was carried out in the same manner once again.

By using a probe specific to the CD8-positive T cells of an HIV-infected person as described in the above, screening (the first) was carried out on 20,000 colonies of the cDNA library of the HIV-infected CD8-positive T cells to pick up 300 positive clones showing an intense signal and 10 colonies in the vicinity thereof, which repeated screening (the second screening) resulted in 22 positive clones showing an intense signal.

**Table 2:**

| Screening with subtracted DNA probe | |
|---|---|
| | No. of positive clones |
| Initial | 20,000 |
| 1st screening | 300 |
| 2nd screening | 22 |

### Example 3:

### Nucleotide sequencing and homology screening for the positive clones (Table 4)

The colonies hybridized with a radioactive-labeled probe were picked up from the replica plate and cultivated. The cells were recovered by centrifugation and lysed to extract plasmid. The cDNA cleaved by digestion with EcoRI was examined for the size by use of agarose electrophoresis (Fig 3). Plasmids containing insert cDNA with 500 bp or more were sequenced by use of a DNA sequencer (supplied by Applied Bio) after chain extension reaction with Sequencing Primer-M4 (supplied by Takara) and RV (supplied by Takara) for the sequences located upstream and downstream of the pUC118 cloning site as well as Dye terminator cycle sequencing FS ready Reaction Kit (supplied by Perkin-Elmer). The resultant sequence was checked for the nucleic acid data base (GenBank).

Table 3 tabulates the homology-screening results and specificity of the positive clones obtained by the screening; plasmids from 22 positive clones were isolated and digested with restriction enzyme EcoRI, followed by agarose gel electrophoresis, leading to the finding that there were insertions having a chain length of 500 to 3,000 bp. Following the Dye terminator method, sequencing was conducted by use of a DNA sequencer, and the resultant sequences were checked for the nucleic acid data base (GenBank), and there were discovered several novel sequences as well as the sequence for human IL-9 Receptor.

**Table 3:**

| Sequence analysis of positive clones | | | | | |
|---|---|---|---|---|---|
| Clone No. | Size (bp) | Homology | % | Location | Pt.CD8 Sp. |
| 1 | 950 | Al repeat | 85 | non code | NT |
| 2 | 1500 | CpG Island/Alu repeat | | | NT |
| 3 | 1500 | Al repeat | | | NT |
| 4 | 500 | None | | | NT |
| 5 | 1500 | Al repeat | 83 | | NT |
| 6 | 500 | None | | | NT |
| 7 | 1400+3000 | None | | | NT |
| 8 | 3500 | Al repeat | | | NT |
| 9 | 500 | None | | | NT |
| 10 | 1400 | IL-9 receptor | 100 | exon 9 | + |
| 11 | 1400 | None | | | NT |
| 12 | 550+600 | IL-9 receptor | 100 | exon 9 | + |
| 13 | 400+500 | human cDNA clone | 100 | unknown | NT |
| 14 | 1500+1400 | None | | | NT |
| 15 | 450 | NT | | | NT |
| 16 | 700 | NT | | | NT |
| 17 | 450+500 | human cDNA clone | 89 | unknown | - |
| 18 | 670 | human cDNA clone | 95 | unknown | - |
| 19 | 450 | human cDNA clone | 95 | unknown | - |
| 20 | 500+600 | None | | | - |
| 21 | 800 | None | | | NT |
| 22 | 1000 | None | | | - |

The RT-PCR method (which involves synthesizing the first-strand cDNA and conducting PCR with the same used as a template) was carried out with mRNAs derived individually from HIV-infected CD8-positive T cells, healthy CD8-positive T cells and CD4-positive T-cells, revealing the band specific to HIV-infected CD8-positive T-cells only when use was made of a primer for a clone being homologous with IL-9R.

### Example 4

### Confirmation of specific expression of mRNA

With HIV-infected CD8-positive T cells, healthy CD8-positive T cells and CD4-positive T cells, individual mRNAs were isolated by use of Quick prep mRNA Micro purification Kit (supplied by Pharmacia), and reverse transcription of mRNAs was conducted with Ready To Go 1st strand synthesis Kit (supplied by Pharmacia) to thereby synthesize the first-strand cDNAs. using the primers (Table 4) upstream and downstream of the in-advance designed and constructed nucleotide sequence, PCR was carried out, with the result that expression of mRNA was confirmed by subsequently done 2.5 % agarose-gel electrophoresis (Fig. 4).

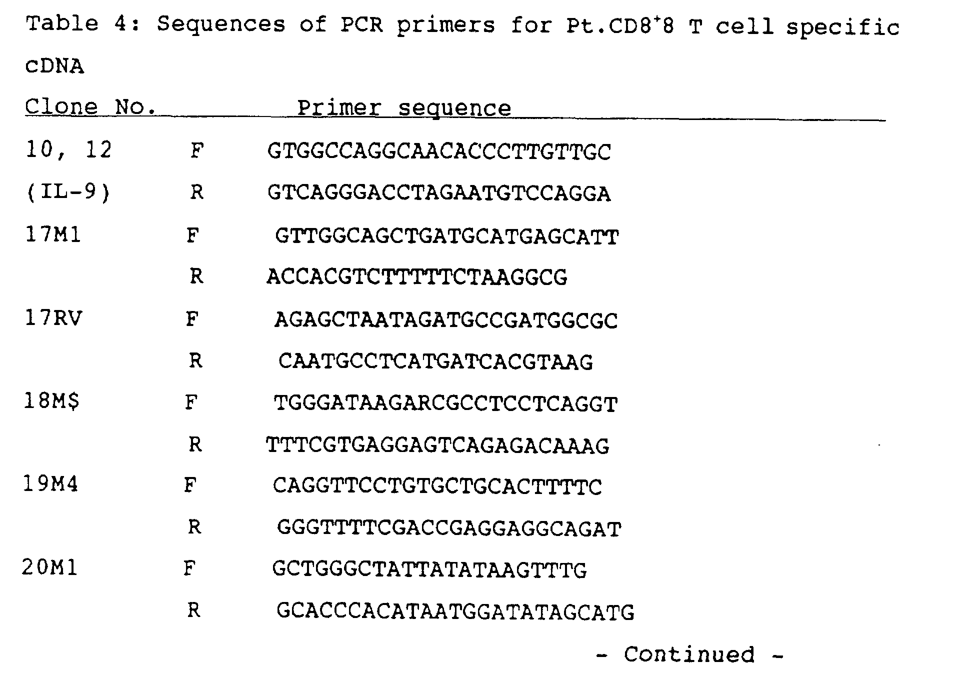

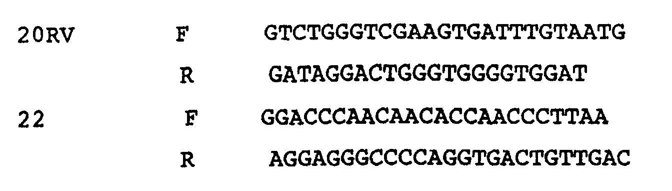

### Example 5

### Establishment of anti-HIV analysis system and assay of anti-HIV activity using CD4-positive T cells transfected with DNA of infectious HIV.

Assay of anti-HIV activity of CD8-positive T cells is based on CD4-positive T cells originated from an HIV-infected person being used as a target cell. Since it is difficult to collect CD4-positive T cells from HIV-1 infected persons under advanced conditions, however, the present inventors have successfully established a method of preparing CD4-positive T cells transfected with DNA of HIV.

Particularly, 1 x 10⁶ of cells were transfected with 1mg of pNL 432 (infectious HIV-1 DNA clone) or pGH 123 (infectious HIV-2 DNA clone) using DEAE-Dextran procedure, followed by incubation on RPMI 1640 10 % FCS in the presence of 5 % of CO₂ for 5 hours at 37°C.

Five hours later, a culture supernatant of infected CD8-positive T cells was added to a transfected cell solution in such a proportion as the added supernatant may occupy one-fourth of the total volume. Furthermore, equine polyclonal anti-IFN-α antibody and recombinant IL-2 were added to the medium to the final concentrations of 50 µ/ml and 0.25 µ/ml, respectively, and the culture supernatant was collected Day 6 to assay the p17 antigen of HIV.

Table 5 tabulates a relationship in Tall-1-CD4 cells between anti-HIV activity of a culture supernatant of CD8-positive T cells from HIV-infected persons and expression of IL-9R

**Table 5:**

| Effects of supernatant of CD⁸T-cell culture from HIV-infected individuals on IL-9 mediated suppression of HIV replication in Tall-1-CD4 cells | | | | |
|---|---|---|---|---|
| Sample Id^{a} | Clinical state^{b} | p17^{a}, pg/ml | Suppression^{d}, % | IL-9 Expression^{e} |
| Neg. control | CD8 supply | 10 | 100 | - |
| Pos. control | | 720 medium | 0 | -? |
| Infected p. | Asymptomatic | 55 | 93.7 | +++ |
| Infected p. | | 1675 | 0 | - |
| Infected p. | Asymptomatic | 405 | 44.4 | ++ |
| Infected p. | | 1900 | 0 | - |
| Infected p. | | 3000 | 0 | - |
| Infected p. | Candida | 3400 | 0 | - |
| Infected p. | Asymptomatic | 1105 | 0 | - |
| Infected p. | Asymptomatic | 920 | 0 | - |
| Infected p. | | 750 | 0 | - |
| Infected p. | Asymptomatic | 650 | 9.9 | - |
| Non-infected p. | | 3000 | 0 | - |
| Non-infected p. | | 1275 H4 | 0 | - |
| Non-infected p. | | 765 H3 | 0 | - |
| Notes: a: Sample; CD8-positive T cells; HIV-infected persons (n = 10) and non-infected persons (n = 3). b: Clinical stage. c: HIV antigen of Tall-1-CD4 cell culture on Day 6 of Incubation. (P17 antigen, pg/ml). d: Suppression in percentage as calculated from negative and positive controls. e: Expression of IL-9R as detected by RT-PCR assay. Relative intensity of expression was in comparison with the expression of G3PDH, or a house keeping gene. | | | | |

## Claims

1. A diagnostic method for detecting HIV infection, consisting of detecting or measuring expression by CD8⁺ T-cell lymphocytes of the T-cell specific interleukin-9 receptor.

2. A method as claimed in Claim 1, consisting of detecting or measuring the production of mRNA coding for IL-9R in a culture of CD8⁺ T-cells.

3. A method as claimed in Claim 1, consisting of detecting or measuring the effect of CD8⁺ T-cell expression products on a test system responsive to IL-9R.

4. A method as claimed in Claim 3, wherein said test system is the IL-9R mediated suppression of HIV replication in T-cells.

5. A method as claimed in Claim 3 or Claim 4, wherein said expression products are introduced into said test system as the supernatant from a culture of CD8⁺ T-cells.

6. A method as claimed in any preceding claim, wherein said method is diagnostic of infection in a long-term asymptomatic HIV infected individual.

## Patentansprüche

1. Diagnostisches Verfahren zur Erkennung einer HIV-Infektion, bestehend aus der Erkennung oder Messung der Expression durch CD8⁺ T-Lymphozyten (T-Zellen-Lymphozyten) des für T-Zellen spezifischen Interleukin-9-Rezeptors.

2. Verfahren nach Anspruch 1, bestehend aus der Erkennung oder Messung der Produktion von mRNA, die in einer Kultur von CD8⁺ T-Zellen für IL-9R kodiert.

3. Verfahren nach Anspruch 1, bestehend aus der Erkennung oder Messung des Effektes von CD8⁺ T-Zellen - Expressionsprodukten auf ein Testsystem, das auf IL-9R antwortet.

4. Verfahren nach Anspruch 3, bei dem das genannte Testsystem die über IL9R vermittelte Suppression der HIV-Replikation (Autoduplikation) in T-Zellen ist.

5. Verfahren nach Anspruch 3 oder 4, bei dem die genannten Expressionsprodukte in das genannte Testsystem in Form des Überstandes aus einer Kultur von CD8⁺ T-Zellen eingeführt werden.

6. Verfahren nach einem der vorstehenden Ansprüche, bei dem das genannte Verfahren die Diagnose einer Infektion bei einem langfristig asymptomatisch durch HIV infizierten Individuum leistet.

## Revendications

1. Méthode de diagnostic pour détecter une infection par HIV, consistant à détecter ou mesurer l'expression par des lymphocytes T CD8+ du récepteur d'inferleukine-9 spécifique des cellules T.

2. Méthode selon la revendication 1, consistant à détecter ou mesurer la production d'ARNm codant IL-9R dans une cellule de cellules T CD8+.

3. Méthode selon la revendication 1, consistant à détecter ou mesurer l'effet de produits d'expression de cellules T CD8+ sur un système de test sensible à IL-9R.

4. Méthode selon la revendication 3, dans laquelle ledit système de test réside dans la suppression, à médiation par IL-9R, de la réplication de VIH dans les cellules T.

5. Méthode selon la revendication 3 ou la revendication 4, dans laquelle lesdits produits d'expression sont introduits dans ledit système de test sous la forme du surnageant provenant d'une culture de cellules T CD8+.

6. Méthode selon l'une quelconque des revendications précédentes, dans laquelle ladite méthode est diagnostique d'une infection chez un individu infecté par VIH asymptomatique sur un long terme.
